(19) Europäisches Patentamt
European Patent Office
Office européen des brevets

(11) Veröffentlichungsnummer: **0 508 269 B1**

# (12) EUROPÄISCHE PATENTSCHRIFT

(45) Veröffentlichungstag der Patentschrift: **01.02.95**

(51) Int. Cl.[6]: **C07C 309/71**, C07C 309/76, C07C 311/16, C07C 323/66, G03F 7/022

(21) Anmeldenummer: **92105467.2**

(22) Anmeldetag: **30.03.92**

## (54) Strahlungsempfindlicher Ester und Verfahren zu dessen Herstellung.

(30) Priorität: **09.04.91 DE 4111443**

(43) Veröffentlichungstag der Anmeldung:
**14.10.92 Patentblatt 92/42**

(45) Bekanntmachung des Hinweises auf die Patenterteilung:
**01.02.95 Patentblatt 95/05**

(84) Benannte Vertragsstaaten:
**BE CH DE FR GB IT LI**

(56) Entgegenhaltungen:
**EP-A- 0 369 219**

**PATENT ABSTRACTS OF JAPAN vol. 12, no. 235 (P-725)(3082) 6. Juli 1988; JP-A-63 027 835**

(73) Patentinhaber: **HOECHST AKTIENGESELLSCHAFT**

**D-65926 Frankfurt (DE)**

(72) Erfinder: **Scheler, Siegfried, Dr.**
**Stormstrasse 5**
**W-6200 Wiesbaden-Naurod (DE)**
Erfinder: **Zahn, Wolfgang, Dr.**
**Draiserweg 7**
**W-6228 Eltville 2 (DE)**
Erfinder: **Schmitt, Axel, Dr.**
**Lärchenweg 5**
**W-6229 Walluf (DE)**
Erfinder: **Buhr, Gerhard, Dr.**
**Am Erdbeerstein 28**
**W-6240 Königstein (DE)**

EP 0 508 269 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die vorliegende Erfindung betrifft neue strahlungsempfindliche Ester aus a) einer Verbindung mit 2 bis 6 aromatischen Hydroxygruppen, b) einer kernsubstituierten o-Naphthochinon-2-diazid-4-sulfonsäure und c) einer nicht weiter substituierten o-Naphthochinon-2-diazid-4- bzw. -5-sulfonsäure und/oder einer nicht strahlungsempfindlichen organischen Säure sowie ein Verfahren zu deren Herstellung.

Die strahlungsempfindliche Schicht üblicher Kopiermaterialien besteht im wesentlichen aus einem Gemisch eines alkalilöslichen Kresol-Formaldehyd-Novolaks mit strahlungsempfindlichen Verbindungen, wie 1,2-Benzo- oder o-Naphthochinon-2-diazidderivaten. Der Novolak ist an sich in wäßrig-alkalischen Lösungen löslich, doch wirken die strahlungsempfindlichen o-Chinondiazidverbindungen als Lösungshemmer. Bei der bildmäßigen Belichtung der Schicht mit aktinischer Strahlung wird die strahlungsempfindliche Diazocarbonyl-Verbindung über ein Keten-Zwischenprodukt in eine Carbonsäure umgewandelt. Die Carbonsäure ist leicht löslich in wäßrig-alkalischer Lösung und verstärkt damit noch die Löslichkeit des Novolaks. Die belichteten Bereiche der Kopierschicht lösen sich in der alkalischen Entwicklerlösung, während die nicht belichteten Bereiche im wesentlichen unverändert erhalten bleiben, so daß auf dem Schichtträger ein positives Reliefbild der Vorlage entsteht.

Das beschriebene Löslichkeitsverhalten läßt sich jedoch auch umkehren. Dazu wird die Aufzeichnungsschicht nach der bildmäßigen Bestrahlung einer Wärmebehandlung unterzogen. In den vom Licht getroffenen Bereichen der Schicht vernetzen dabei die Harzmoleküle der Schicht. Dieser als "Härtung" bezeichnete Vorgang setzt in der Regel das Vorhandensein eines "Vernetzers" voraus, der unter dem Einfluß der Säure, die bei der Belichtung aus dem o-Chinondiazid entstanden ist, während der Wärmebehandlung die Vernetzung und damit die Härtung bewirkt. Beim Härten wird auf Temperaturen unterhalb der Zersetzungstemperatur des o-Chinondiazids erwärmt. Das Erwärmen kann durch Bestrahlen, Einbringen in einen Strom heißen Gases, Kontakt mit erwärmten Flächen, z. B. mit beheizten Walzen, oder durch Tauchen in ein erwärmtes Bad einer inerten Flüssigkeit, z. B. Wasser, erfolgen. Die Temperatur liegt im allgemeinen zwischen 100 und 150 °C, vorzugsweise zwischen 90 und 130 °C.

Wirksame Vernetzer sind allgemein Verbindungen, die unter den beschriebenen Säure- und Temperaturverhältnissen leicht ein Carboniumion bilden. Beispiele hierfür sind die Hexamethylolmelaminether gemäß DE-A 33 25 022 (= US-A 4 581 321) sowie die in der EP-A 0 212 482 beschriebenen Verbindungen, wie 1,4-Bis-hydroxymethylbenzol und 4,4'-Bis-methoxymethyldiphenylether, in denen zwei oder mehr aromatisch gebundene Hydroxymethyl- oder Alkoxymethylgruppen enthalten sind. Als Vernetzer ist auch 2,6-Bis-hydroxymethyl-4-methyl-phenol gemäß US-A 4 404 272 bekannt.

Nach der Wärmebehandlung wird die Photoresistschicht in der Regel einer vollflächigen Belichtung ("Flutbelichtung") unterworfen, um die noch strahlungsempfindlichen Schichtbereiche vollständig alkalilöslich zu machen.

Die Flutbelichtung erfolgt im allgemeinen mit der gleichen Lichtquelle, die auch für die Bildbelichtung verwendet wurde.

Die Entwicklung im Anschluß an die Flutbelichtung wird im allgemeinen mit einer der wäßrig-alkalischen Lösungen durchgeführt, die auch zur Entwicklung eines positiv arbeitenden Photoresists verwendet werden. Dies sind z. B. wäßrige Lösungen von Natriumhydroxid, Tetramethylammoniumhydroxid, Trimethyl-(hydroxyethyl)ammoniumhydroxid, Alkaliphosphaten, -silikaten oder -carbonaten, die Netzmittel oder kleinere Mengen organischer Losemittel enthalten können. Bei der Entwicklung werden die bei der ursprünglichen Bildbelichtung nicht vom Licht getroffenen Schichtbereiche ausgewaschen, so daß ein negatives Resistbild der Vorlage erhalten wird.

In den meisten Fällen wird das belichtete und entwickelte Resistmaterial dann mit einem Ätzmittel behandelt, wobei das Ätzmittel nur in den Nichtbildbereichen auf den Schichtträger einwirken kann. Auf diese Weise wird auf dem Schichtträger im Falle einer positiv arbeitenden Kopierschicht ein negatives Ätzbild und im Falle einer negativ arbeitenden Kopierschicht ein positives Ätzbild erzeugt.

Das nach den beschriebenen Verfahren auf dem Schichtträger erzeugte positive bzw. negative Reliefbild der Kopierschicht eignet sich für verschiedene Anwendungszwecke, u. a. als Belichtungsmaske oder als Bild bei der Herstellung von Halbleiterbauelementen in der Mikroelektronik, als Druckform für den Hoch-, Tief- oder Flachdruck sowie für die Herstellung von Nickelrotationszylindern in einem galvanischen Verfahren.

Die kommerzielle Eignung einer Kopierschicht, z. B. einer Photoresistschicht, wird u. a. nach der Strahlungsempfindlichkeit, dem Entwicklungs- und Bildkontrast, der Auflösung und der Haftung auf dem Schichtträger beurteilt.

Eine hohe strahlungsempfindlichkeit des Gemisches ist bei der Fertigung mikroelektronischer Schaltkreise oder Bauelemente ein wesentlicher Faktor, vor allem bei der sog. "in-line"-Verarbeitung von Wafern,

bei der der Durchsatz an Scheiben von dem am längsten dauernden Prozeßschritt bestimmt wird. Bei den bisher notwendigen relativ langen Belichtungszeiten ist der Durchsatz am Belichtungsgerät der limitierende Faktor. Vor allem bei monochromatischer Bestrahlung und bei Bestrahlung mit kürzerwelligem, aktinischem Licht sind die Taktzeiten am Belichtungsgerät zu lang, was eine zu geringe Produktionsgeschwindigkeit zur Folge hat.

Die Resistauflösung betrifft die Fähigkeit eines Photoresistsystems, auch die feinsten Linien und Zwischenräume einer für die Belichtung verwendeten Maske wiederzugeben, wobei die belichteten und entwickelten Bereiche ein hohes Maß an Kantensteilheit und -schärfe aufweisen sollen.

In vielen technischen Einsatzgebieten, insbesondere bei der Herstellung von Halbleiterelementen in der Mikroelektronik, muß der Photoresist eine besonders hohe Auflösung erreichen, denn es müssen sehr kleine Linien- und Zwischenraumbreiten (< 1 µm) wiedergegeben werden. Die Fähigkeit, kleinste Details in der Größenordnung von 1 µm und darunter wiederzugeben, ist für die großtechnische Herstellung von integrierten Schaltungen auf Siliciumchips und ähnlichen Bauteilen von größter Bedeutung. Die Integrationsdichte auf einem solchen Chip läßt sich bei Verwendung photographischer Verfahren durch eine Steigerung des Auflösungsvermögens des Photoresists erhöhen.

Es ist bekannt, daß die Auflösung eines Photoresists steigt, wenn darin photoaktive Verbindungen mit mehreren strahlungsempfindlichen Resten in einem Molekül vorhanden sind, da dann der strahlungsempfindliche Anteil im Gemisch erhöht ist. [P. Trefonas III and B. K. Daniels, "New Principle for Image Enhancement in Single Layer Positive Photoresists", SPIE Advances in Resist Technology and Processing IV, 771 (1987), 194-210; P. Trefonas III, B. K. Daniels and R. L. Fischer, "Photoresist Design for Submicron Optical Lithography: Application of Polyphotolysis", Solid State Technology 30 (1987) 131-137].

Bei Verwendung der in den EP-A 0 244 762 und 0 244 763 genannten Mischester ist bereits eine verbesserte Auflösung festzustellen. In der DE-A 38 37 500 werden die überlegenen Eigenschaften von kernsubstituierten Naphthochinondiazidderivaten in lithographischen Anwendungen gezeigt. Ester von gegebenenfalls substituierten o-Naphthochinon-2-diazid-4-oder -5-sulfonsäuren mit Verbindungen, die drei oder mehr aromatische Hydroxygruppen besitzen, weisen jedoch häufig eine zu geringe Löslichkeit in den üblichen Lösemitteln auf, was dazu führt, daß die mit diesen Estern hergestellten Resists noch keine ausreichende Auflösung erreichen.

Aufgabe der vorliegenden Erfindung war es daher, neue Verbindungen aus substituierten o-Naphthochinon-2-diaziden und Verbindungen mit mindestens zwei und mehr aromatischen Hydroxygruppen zu entwickeln, die im Vergleich zu bekannten Verbindungen eine gesteigerte Löslichkeit in den üblichen Lösemitteln wie auch in Systemen aus Lösemitteln und Bindemitteln (Harz) aufweisen.

Die Aufgabe wurde überraschenderweise gelöst durch einen strahlungsempfindlichen Ester aus

a) einer Verbindung mit zwei bis sechs aromatischen Hydroxygruppen (im Folgenden als aromatische Polyhydroxyverbindung bezeichnet),

b) einer kernsubstituierten o-Naphthochinon-2-diazid-4-sulfonsäure (Diazoverbindung $D_1$) und

c) einer nicht weiter substituierten o-Naphthochinon-2-diazid-4- bzw. -5-sulfonsäure (Diazoverbindung $D_2$) und/oder einer nicht strahlungsempfindlichen organischen Säure (Verbindung $D_0$),

wobei das molare Verhältnis von $D_1$:($D_2$ und/oder $D_0$) zwischen 0,1:1 und 39:1 liegt.

Geeignete aromatische Polyhydroxyverbindungen sind insbesondere Verbindungen der allgemeinen Formel I

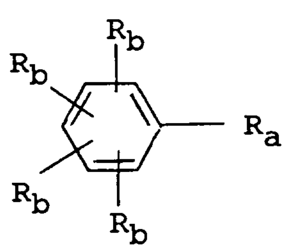

(I)

wobei $R_a$ = -H, -X-$R_c$ oder

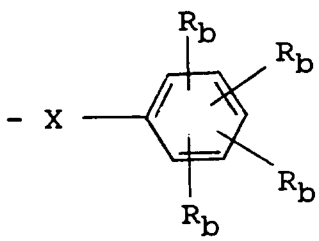

darstellt, worin

$R_b$ Wasserstoff- oder Halogenatome, Hydroxy- oder ($C_1$-$C_6$)Alkylgruppen bedeuten, mit der Maßgabe, daß mindestens zwei und höchstens sechs der $R_b$-Gruppen Hydroxygruppen und die Reste $R_b$ untereinander gleich oder verschieden sind,

X eine Einfachbindung, -O-, -S-, $-SO_2-$, -CO-, $-CO-CH_2-$, $-CO-CH_2-CH_2-$ $-CO-CH_2-CO-$, $-CO-CH_2-CH_2-CO-$, -CO-O-, $-CO-O-CH_2-$, $-CO-O-CH_2-CH_2-$, $-CH_2-CO-O-CH_2-$, $-CH_2-CO-O-CH_2-CH_2-$, $-CH_2-$, $-CH_2-CH_2-$, $-(CH_2)_3-$, $-C(CH_3)_2-$, $-C(CF_3)_2-$, $-CH_2-CH_2-Y$, $-CH_2-CH_2-CH_2-Y$, $-C(CH_3)-CH_2-Y$, $-C(CH_3)-CH_2-CH_2-Y$, wobei $CH_2$-Gruppen durch -O-, H-Atome durch Substituenten ersetzt sein können,

Y ein Wasserstoffatom oder eine gegebenenfalls substituierte Alkoxy-, Carboxy-, Alkoxycarbonyl-, Alkoxyalkoxycarbonyl- oder Arylgruppe und

$R_c$ ein Wasserstoffatom oder eine gegebenenfalls substituierte Alkyl- oder Arylgruppe bedeutet.

Geeignete Polyhydroxyverbindungen sind daneben auch Verbindungen der allgemeinen Formel II

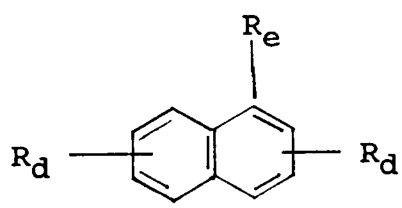

(II)

worin

$R_e$ ein Wasserstoffatom oder eine gegebenenfalls substituierte Naphthalin-1-ylmethylgruppe

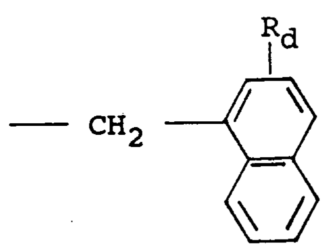

bedeutet, wobei

$R_d$ ein Wasserstoffatom oder eine Hydroxygruppe darstellt, mit der Maßgabe, daß mindestens zwei der $R_d$-Gruppen Hydroxygruppen sind.

Geeignete Polyhydroxyverbindungen sind schließlich auch solche der allgemeinen Formel III

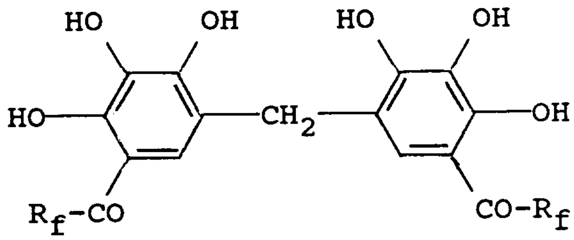

(III)

worin

$R_f$ ein Wasserstoffatom oder eine gegebenenfalls substituierte Alkyl-, Alkoxy- oder Arylgruppe darstellt.

Kernsubstituierte o-Naphthochinon-2-diazid-4-sulfonsäuren (Diazoverbindungen $D_1$) sind Verbindungen der allgemeinen Formel IV

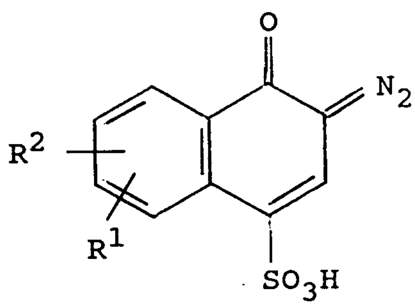

(IV)

worin

$R^1$ und $R^2$ gleich oder verschieden sind und ein Wasserstoffatom, eine Alkyl-, Alkoxy- oder Alkylmercaptogruppe, deren C-Ketten durch Sauerstoffatome unterbrochen sein können, eine Acylamino-, Alkoxycarbonyl-, N-Alkylsulfamoyl oder N,N-Dialkyl-sulfamoylgruppe bedeuten, mit der Maßgabe, daß

$R^1$ und $R^2$ nicht gleichzeitig Wasserstoff sind.

Bevorzugte Verbindungen der Formel IV sind solche, in denen

$R^1$ Wasserstoff und

$R^2$ eine Alkyl-, Alkylether- oder Alkylmercaptogruppe, deren C-Ketten durch Ethersauerstoffatome unterbrochen sein können,

bedeuten. Besonders bevorzugt ist die 7-Methoxy-1,2-naphthochinon-2-diazid-4-sulfonsäure.

Besonders geeignete Diazoverbindungen $D_2$ sind o-Naphthochinon-2-diazid-4- und -5-sulfonsäuren der allgemeinen Formel V

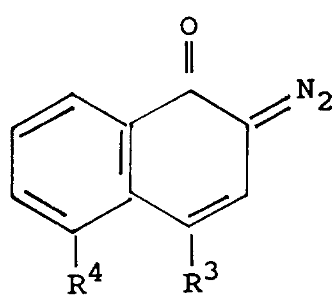

(V)

worin

$R^3$ und $R^4$ verschieden sind und ein Wasserstoffatom oder eine Sulfogruppe bedeuten.

Die Verbindungen $D_0$ sind nicht strahlungsempfindliche organische Säuren der allgemeinen Formel VI

$R^5$ - Z - OH (VI)

worin

$R^5$ eine gesättigte oder ungesättigte, geradkettige oder verzweigte ($C_1$-$C_{25}$)Alkyl-, ($C_1$-$C_{25}$)Alkoxy- oder ($C_1$-$C_{25}$)Alkylmercaptogruppe, deren C-Ketten durch Sauerstoffatome unterbrochen oder die substituiert sein können, eine gegebenenfalls substituierte ($C_6$-$C_{14}$)Aryl-, ($C_6$-$C_{14}$)Aralkyl- oder ($C_6$-$C_{14}$)Cycloalkylgruppe und

Z eine Carbonyl- oder Sulfonylgruppe

bedeuten.

Im folgenden werden einige besonders geeignete aromatische Polyhydroxyverbindungen der allgemeinen Formel I aufgezählt:
Polyhydroxybenzole, wie 1,3-Dihydroxybenzol, 1,4-Dihydroxybenzol, 1,2,3-Trihydroxybenzol, 1,2,4-Trihydroxybenzol, 1,3,5-Trihydroxybenzol usw.;
Dihydroxybenzophenone, wie 2,2'-Dihydroxybenzophenon, 2,3'-Dihydroxybenzophenon, 2,4-Dihydroxybenzophenon, 2,4'-Dihydroxybenzophenon, 2,5-Dihydroxybenzophenon, 3,3'-Dihydroxybenzophenon, 4,4'-Dihydroxybenzophenon usw.;
Trihydroxybenzophenone, wie 2,6,2'-Trihydroxybenzophenon, 2,3,4-Trihydroxybenzophenon, 2,4,4'-Trihydroxybenzophenon, 2,4,6-Trihydroxybenzophenon, 3,4,5-Trihydroxybenzophenon usw.;
Tetrahydroxybenzophenone, wie 2,3,4,2'-Tetrahydroxybenzophenon, 2,4,2',4'-Tetrahydroxybenzophenon, 2,4,2',6'-Tetrahydroxybenzophenon, 2,5,2',6'-Tetrahydroxybenzophenon, 2,3,4,4'-Tetrahydroxybenzophenon, 2,4,3',4'-Tetrahydroxybenzophenon, 2,4,6,3'-Tetrahydroxybenzophenon, 2,4,6,4'-Tetrahydroxybenzophenon, 3,4,3',4'-Tetrahydroxybenzophenon usw.;
Pentahydrorybenzophenone, wie 2,3,4,2',4'-Pentahydroxybenzophenon;
Hexahydroxybenzophenone, wie 2,3,4,3',4',5'-Hexahydroxybenzophenon;
Di- und Trihydroxyphenyl-alkyl-ketone, wie 2,4-Dihydroxyphenyl-alkyl-keton,2,5-Dihydroxyphenyl-alkyl-keton, 3,4-Dihydroxyphenyl-alkyl-keton, 3,5-Dihydroxyphenyl-alkyl-keton, 2,3,4-Trihydroxyphenyl-alkyl-keton, 3,4,5-Trihydroxyphenyl-alkyl-keton, 2,4,6-Trihydroxyphenyl-alkyl-keton usw., wobei der Alkylteil vorzugsweise ($C_1$-$C_{12}$)Alkylgruppen bezeichnet, die gegebenenfalls verzweigt sind, wie Methyl, Ethyl, Butyl, n-Hexyl, Heptyl, Decyl, Dodecyl usw.;
Di- und Trihydroxyphenyl-aralkyl-ketone, Di-, Tri- und Tetrahydroxydiphenyle, Dihydroxydiphenylether, Dihydroxydibenzylether und Di(hydroxydiphenyl)alkane, deren Alkanteil sich vorzugsweise von niederen Alkanen, wie Methan, Ethan, Propan usw. ableitet. Der Alkanteil kann auch substituiert sein, wobei eine besonders bevorzugte Verbindung dieser Art 4,4-Bis-(4-hydroxyphenyl)-valeriansäure-(2-ethoxyethyl)ester ist;
Hydroxybenzoesäuren und deren Ester, wie Di- und Trihydroxybenzoesäure, Di- und Trihydroxybenzoesäurealkylester, wobei die Alkylgruppe vorzugsweise 1 bis 12 C-Atome, insbesondere 1 bis 8 C-Atome, besitzt, insbesondere 2,4-, 2,5-, 3,4- und 3,5-Dihydroxybenzoesäure-n-butylester, 2,4-Dihydroxybenzoesäure-2,4,4-Trimethylpentyl-ester usw.; Di- und Trihydroxybenzoesäurephenylester, Di-, Tri- und Tetrahydroxydiphenylsulfide, wie 4,4'-Dihydroxydiphenylsulfid; Dihydroxydiphenylsulfone, Di- und Trihydroxyphenylnaphthyl-ketone, wie 2,3,4-Trihydroxyphenyl-naphthalen-1-yl-keton und ähnliche Verbindungen.

Zu den Verbindungen der allgemeinen Formel I, bei denen wenigstens eine $R_b$-Gruppe ein Halogenatom oder eine niedere Alkylgruppe darstellt, gehören beispielsweise 2,4-Dihydroxy-3,5-dibrombenzophenon, 5-Brom-2,4-dihydroxybenzoesäure und deren Ester, 2,4,2',4'-Tetrahydroxy-3,5,3',5'-tetrabromdiphenyl, 4,4'-Dihydroxy-2,2'-dimethyl-5,5'-di.tert.-butyldiphenyl, 4,4'-Dihydroxy-2,2'-dimethyl-5,5'-di.tert.-butyldiphenyl-sulfid, 2,4,2',4'-Tetrahydroxy-3,5,3',5'-tetrabromdiphenylsulfon und ähnliche.

Als aromatische Polyhydroxyverbindungen der allgemeinen Formel I werden die Tri-, Tetra- und Hexahydroxybenzophenone sowie die Trihydroxyphenyl-alkyl-ketone bevorzugt eingesetzt.

Zu den Phenolverbindungen der allgemeinen Formel II gehören vorzugsweise folgende Verbindungen: Dihydroxynaphthaline, wie 1,2-, 1,4-, 1,5-, 1,6-, 1,7-, 1,8-, 2,3-, 2,6- und 2,7-Dihydroxy-naphthalin, Dihydroxydinaphthylmethane, wie 2,2'-Dihydroxydinaphthylmethan.

Die Dihydroxynaphthaline werden bevorzugt eingesetzt. Die Hydroxylgruppen der Dihydroxynaphthaline können sich dabei sowohl sämtlich an einem Kern, als auch vorteilhaft an beiden Kernen des Naphthalinmoleküls befinden.

Verbindungen der allgemeinen Formel III sind vorzugsweise Bis-(3-benzoyl-4,5,6-trihydroxyphenyl)-methan, Bis-(3-acetyl-4,5,6-trihydroxyphenyl)-methan, Bis-(3-propionyl-4,5,6-trihydroxyphenyl)-methan, Bis-(3-butyryl-4,5,6-trihydroxyphenyl)-methan, Bis-(3-hexanoyl-4,5,6-trihydroxyphenyl)-methan, Bis-(3-heptanoyl-4,5,6-trihydroxyphenyl)-methan, Bis-(3-decanoyl-4,5,6-trihydroxyphenyl)-methan und Bis-(3-octadecanoyl-4,5,6-trihydroxyphenyl)-methan.

Die Veresterung erfolgt im allgemeinen mit geeigneten aktivierten Derivaten der Naphthochinondiazidsulfonsäuren, vor allem den Säurehalogeniden, insbesondere den Säurechloriden. Geeignete Derivate von Diazoverbindungen $D_1$ der allgemeinen Formel IV sind z. B. **(A)** 5-Methoxy-1,2-naphthochinon-2-diazid-4-sulfonylchlorid, **(B)** 6-Methoxy-1,2-naphthochinon-2-diazid-4-sulfonylchlorid, **(C)** 7-Methoxy-1,2-naphthochinon-2-diazid-4-sulfonylchlorid, **(D)** 8-Methoxy-1,2-naphthochinon-2-diazid-4-sulfonylchlorid, **(E)** 5-Methyl-1,2-naphthochinon-2-diazid-4-sulfonylchlorid, **(F)** 6,7-Dimethyl-1,2-naphthochinon-2-diazid-4-sulfonylchlorid, **(G)** 7-Acetylamino-1,2-naphthochinon-2-diazid-4-sulfonylchlorid, **(H)** 7-Methylmercapto-1,2-naphthochinon-2-diazid-4-sulfonylchlorid, **(I)** 7-Dimethylsulfamoyl-1,2-naphthochinon-2-diazid-4-sulfonylchlorid, **(J)** 6-Brom-1,2-naphthochinon-2-diazid-4-sulfonylchlorid, **(K)** 6-Methoxycarbonyl-1,2-naphthochinon-2-diazid-4-sulfonylchlorid.

Besonders bevorzugt ist 7-Methoxy-1,2-naphthochinon-2-diazid-4-sulfonylchlorid, dessen Herstellung in der DE-A 38 37 500 sowie in den nicht vorveröffentlichten deutschen Patentanmeldungen P 39 26 774.1 und P 39 26 776.8 angegeben ist.

Geeignete Derivate von Diazoverbindungen $D_2$ der allgemeinen Formel V sind z. B. **(L)** o-Naphthochinon-2-diazid-4-sulfonylchlorid und **(M)** o-Naphthochinon-2-diazid-5-sulfonylchlorid.

Geeignete Derivate von organischen, nicht strahlungsempfindlichen Säuren der allgemeinen Formel VI sind beispielsweise Acylhalogenide, wie **(N)** Acetyl-, Propionyl-, Butyryl-, Isobutyryl-, Valeryl-, Isovaleryl-, Pivaloyl-, Caproyl-, Capryloyl-, Caprinoylchlorid, **(O)** Lauroyl-, Myristoyl-, Palmitoylchlorid, **(P)** Stearoyl-, Cerotoyl- (= Hexacosanoyl-), Acryloyl-, Oleoyl-, Linoloyl-, Docos-11-enoyl-, Tetracos15c-enoylchlorid, **(O)** Benzoyl- und Naphthoylchlorid, **(R)** 2-Ethyl-hexanoyl-, 7-Methyl-octanoyl-, 7,7-Dimethyl-octanoylchlorid, **(S)** 2-Chlor-propionyl-, 4-Chlorbutyrylchlorid, **(ST)** Methoxyessigsäurechlorid, Chlorameisensäure-methylester, -ethylester, -propylester, -butylester, **(T)** -2-ethylhexylester, -octylester, -hexadecylester, -methoxyethylester, Alkansulfonylhalogenide, wie **(U)** Methan-, Ethan-, Propan-, n-Butan- und Dodecan-sulfonylchlorid; Arylsulfonylhalogenide, wie **(V)** Benzol-, **(W)** Toluol- und Naphthalin-sulfonylchlorid.

Bevorzugt werden Alkanoyl- und Alkansulfonyl-halogenide mit 1 bis 16 C-Atomen sowie Aroyl- und Arylsulfonyl-halogenide mit 6 bis 8 C-Atomen. Die Säurehalogenide können gegebenenfalls substituiert sein.

Die Herstellung der Halogenide der Säuren der allgemeinen Formeln V und VI erfolgt analog zu literaturbekannten Verfahren. Gegenüber den nur mit der Diazoverbindung $D_1$ oder der Diazoverbindung $D_2$ hergestellten Estern zeichnen sich die erfindungsgemäßen Mischester durch eine überraschend höhere Löslichkeit in den für strahlungsempfindliche Gemische üblichen Lösemitteln bzw. Lösemittelgemischen aus. Die lithographischen Eigenschaften der erfindungsgemäßen Mischester, besonders solcher, die durch Kondensation von aromatischen Polyhydroxyverbindungen mit einem Gemisch aus den Diazoverbindungen $D_1$ und $D_2$ im Molverhältnis von 1,5:1 und 9:1 erhalten werden, sind besser als die der bekannten Ester auf der Basis von o-Naphthochinon-2-diazid-4- und -5-sulfonsäure. Aufgrund ihrer günstigen Absorptionscharakteristik sind die neuen Mischester sowohl für i-line- als auch für g-line-Belichtung gleichermaßen gut geeignet.

Die Erfindung betrifft auch ein Verfahren zur Herstellung der erfindungsgemäßen Mischester, bei dem eine der aromatischen Polyhydroxyverbindungen der allgemeinen Formeln I bis III in einem polaren organischen Lösemittel in Anwesenheit einer basischen Verbindung mit dem Gemisch aus dem o-Naphthochinon-2-diazid-4-sulfonylchlorid ($D_1$) und dem o-Naphthochinon-2-diazid-4- bzw. - 5-sulfonylchlorid ($D_2$) oder dem nicht strahlungsempfindlichen organischen Säurechlorid ($D_0$) kondensiert wird, wobei das Molverhältnis von $D_1$:($D_2$ und/oder $D_0$) in dem Gemisch zwischen 0,1:1 und 39:1, bevorzugt zwischen 0,25:1 und 19:1, insbesondere zwischen 1,5:1 und 9:1, liegt und die Kondensationsprodukte isoliert und gegebenenfalls gereinigt werden. Beispielsweise wird 1 mol 2,3,4,4'-Tetrahydroxybenzophenon mit einem Gemisch im Mischungsverhältnis 9:1 aus 3,6 mol Sulfonylchlorid $D_1$ und 0,4 mol Sulfonylchlorid $D_2$ kondensiert. Die erhaltene strahlungsempfindliche Verbindung umfaßt Einheiten der allgemeinen Formel VII

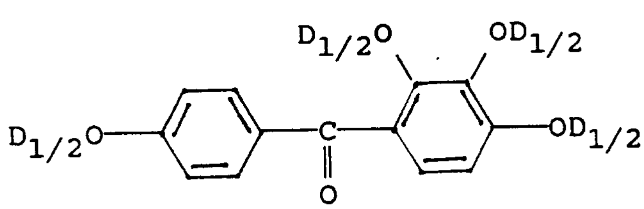

(VII)

in der

$D_1$ und $D_2$ unabhängig voneinander jeweils eine o-Naphthochinon-2-diazid-4-sulfonylgruppe der Verbindung $D_1$ oder eine o-Naphthochinon-2-diazid-4- bzw. -5-sulfonylgruppe der Verbindung $D_2$ bedeuten.

Die aromatische Polyhydroxyverbindung der allgemeinen Formeln I bis III wird vorzugsweise mit etwa der stöchiometrischen Menge des Gemisches aus den Diazosulfonylchloriden $D_1$ und $D_2$ oder dem Diazosulfonylchlorid $D_1$ und dem organischen Säurechlorid $D_0$ umgesetzt. Die Polyhydroxyverbindungen müssen jedoch nicht vollständig verestert sein; es können auch kleinere als stöchiometrische Mengen an Diazosulfonylchlorid $D_1$, $D_2$ oder Säurechlorid $D_0$ für die Kondensation mit der Polyhydroxyverbindung eingesetzt werden.

Die Gesamtmenge der mit den Polyhydroxyverbindungen umgesetzten Sulfonylchloride $D_1$ und $D_2$ bzw. oder $D_0$ sollte so bemessen sein, daß eine strahlungsempfindliche Verbindung erhalten wird, die eine ausreichende Löslichkeit des strahlungsempfindlichen Gemisches im alkalischen Medium nach dem Belichten gewährleistet.

Herstellung der strahlungsempfindlichen Kondensationsprodukte

Verfahren zur Herstellung-von strahlungsempfindlichen Verbindungen auf der Basis von Naphthochinondiaziden werden z. B. in den US-A 3 046 118, 3 106 645, 4 397 937 sowie in den DE-A 38 37 500, 39 26 774 und 39 26 776 beschrieben.

Die erfindungsgemäßen strahlungsempfindlichen Verbindungen werden durch Kondensation der o-Naphthochinon-2-diazidsulfonylchloride $D_1$ und $D_2$ und bzw. oder des nicht strahlungsempfindlichen organischen Säurechlorids $D_0$ mit den Polyhydroxyverbindungen der allgemeinen Formeln I bis III in Gegenwart einer säurebindenden Substanz hergestellt. Die so erhaltenen strahlungsempfindlichen Verbindungen können gegebenenfalls noch gereinigt werden.

Für die Durchführung der Kondensationsreaktion geeignete Lösemittel sind beispielsweise Aceton, Dioxan, Tetrahydrofuran, Acetonitril, Methylenchlorid, Pyridin usw..

Die säurebindende Substanz kann anorganisch sein, wie Natriumcarbonat oder Natriumhydrogencarbonat, oder organisch, z. B. das Natriumsalz einer schwachen organischen Säure, wie Natriumacetat, ein tertiäres Amin, wie Triethylamin, N-Methylmorpholin, 1,4-Diazobicyclo[2.2.2 ]octan (Dabco$^{(R)}$) oder Pyridin bzw. ein Pyridinderivat.

Beispiel 1

Herstellung eines mit den o-Naphthochinon-2-diazid-sulfonylchloriden $D_1$ und $D_2$ im Molverhältnis 4,54:1 kondensierten 2,3,4,4'-Tetrahydroxybenzophenons:

53,01 g (0,2155 mol) 2,3,4,4'-Tetrahydroxybenzophenon und 178,43 g (1,767 mol) N-Methylmorpholin werden in 4,095 l Acetonitril gelöst. In die auf ca. 22 bis 23 °C abgekühlte orange Lösung werden unter Rühren insgesamt 41,81 g (0,1557 mol) festes o-Naphthochinon-2-diazid-5-sulfonylchlorid ($D_2$) in drei Portionen von jeweils 18,68 g, 13,40 g und 9,76 g in zeitlichen Abständen von 2 min eingetragen; die braun-orange Lösung wird noch 79 min nachgerührt. Anschließend werden auf die gleiche Weise insgesamt 211,19 g (0,7075 mol) 7-Methoxy-1,2-naphthochinon-2-diazid-4-sulfonylchlorid ($D_1$) in drei Portionen von 129,9 g, 52,36 g und 28,93 g in zeitlichen Abständen von 10 min zudosiert. Nach Zugabe der zweiten Portion des Sulfonylchlorids $D_1$ beginnt die Abscheidung eines feinen, kristallinen Niederschlags, nach Zugabe der dritten Portion die Abscheidung eines braunen, zähflüssigen Öls. Das Reaktionsgemisch wird noch 3 h bei 25 bis 28 °C nachgerührt und anschließend die flüssige Reaktionsphase abdekantiert. Das an der Innenwand des Reaktionsgefäßes haftende braune, zähe Öl wird in 3 l Aceton gelöst und zu der abdekantierten Reaktionslösung gegeben. Die vereinigten dunkelbraunen Lösungen werden anschließend in 43,86 l eines Gemisches aus Wasser und 37%iger Salzsäure (50:1) unter Rühren eingetropft; die hellgelbe Suspension wird nach 24 h Standzeit bei 22 bis 23 °C abgesaugt, mit 1 l Wasser nachgespült und abgepreßt. Das noch nutschenfeuchte, gelbe Reaktionsprodukt wird anschließend in 6,456 l Aceton gelöst, mit 21,6 g Aktivkohle behandelt und nach Abfiltrieren der Aktivkohle das erhaltene klare, orange Filtrat innerhalb von ca. 90 min in 32,68 l eines Gemisches aus Wasser und 37%iger wäßriger Salzsäure (75:1) unter gutem Rühren eingetropft. Die hellgelbe Suspension wird noch 1 h bei 22 bis 23 °C nachgerührt, anschließend 24 h bei dieser Temperatur stehengelassen und dann das hellgelbe Reaktionsprodukt abgesaugt, dieses mit 2 l Wasser nachgespült, gut abgepreßt und dann im Vakuumtrockenschrank bei 40 °C und 450 Torr während 24 h getrocknet.

Ausbeute: 252 g hellgelbes Pulver, entsprechend 92,13 % d. Th.

Nach dem vorstehend beschriebenen Verfahren wurden eine Reihe von erfindungsgemäßen Kondensationsprodukten hergestellt, in denen die Molverhältnisse der o-Naphthochinon-2-diazidsulfonylchloride $D_1$ und $D_2$ bzw. des o-Naphthochinon-2-diazidsulfonylchlorids $D_1$ und des nicht strahlungsempfindlichen organischen Säurechlorids $D_0$ entsprechend der Tabelle 1 geändert wurden.

Für Vergleichszwecke in den erfindungsgemäßen Gemischen und Aufzeichnungsmaterialien wurden Kondensationsprodukte aus 2,3,4,4'-Tetrahydroxybenzophenon und nur einer der folgenden o-Naphthochinon-2-diazid-sulfonylchloride $D_1$ und $D_2$ im Molverhältnis 1:4 hergestellt:

($D_1$) 7-Methoxy-1,2-naphthochinon-2-diazid-4-sulfonylchlorid und ($D_2$)o-Naphthochinon-2-diazid-4- bzw -5-sulfonylchlorid

Tabelle 1

| Nr. | POLYHYDROXYVERBINDUNGEN | | | | | ORGANISCHE SÄUREHALOGENIDE | | | | | | MOLVERHÄLTNIS | | |
|---|---|---|---|---|---|---|---|---|---|---|---|---|---|---|
| | DHPVE[1]) | TOB[2]) | THB[3]) | PHB[4]) | HHB[5]) | LSH[6]) | | | | NLSH[7]) | | $D_1/D_2$ | $D_1/D_0$ | VG[8]) (%) |
| | MOL | | | | | $D_1$ | $Mol_{D_1}$ | $D_2$ | $Mol_{D_2}$ | $D_0$ | $Mol_{D_0}$ | | | |
| 1 | | 1 | | | | C | 0,4 | M | 3,6 | | | 0,11 : 1 | | 100 |
| 2 | | | | | 1 | C | 1,0 | M | 5,0 | | | 0,50 : 1 | | 100 |
| 3 | | | | 1 | | C | 1,0 | M | 4,0 | | | 0,25 : 1 | | 100 |
| 4 | | 1 | | | | C | 0,6 | M | 2,4 | | | 0,25 : 1 | | 100 |
| 5 | | | 1 | | | C | 0,8 | M | 3,2 | | | 0,25 : 1 | | 100 |
| 5 a | | | 1 | | | C | 1,0 | M | 3,0 | | | 0,33 : 1 | | 100 |
| 6 | | | 1 | | | C | 0,8 | M | 3,0 | | | 0,26 : 1 | | 95 |
| 7 | | | | 1 | | C | 1,0 | M | 3,0 | | | 0,33 : 1 | | 80 |
| 8 | | | | | 1 | C | 1,0 | M | 3,0 | | | 0,33 : 1 | | 66,66 |
| 9 | | | 1 | | | C | 2,0 | L | 2,0 | | | 1,00 : 1 | | 100 |
| 9 a | | | 1 | | | C | 3,5 | L | 0,5 | | | 7,00 : 1 | | 100 |
| 10 | | 1 | | | | D | 1,8 | M | 1,2 | | | 1,50 : 1 | | 100 |
| 11 | | | | 1 | | A | 1,8 | M | 1,2 | | | 1,50 : 1 | | 100 |
| 12 | | | 1 | | | C | 2,4 | M | 1,6 | | | 1,50 : 1 | | 100 |
| 13 | | 1 | | | | E | 1,8 | L | 0,9 | | | 2,00 : 1 | | 90 |
| 14 | | | 1 | | | B | 2,4 | M | 0,6 | | | 4,00 : 1 | | 100 |
| 15 | | | 1 | | | C | 3,3 | M | 0,7 | | | 4,71 : 1 | | 100 |
| 16 | | | | | 1 | C | 5,0 | | | P | 1,0 | | 5,00 : 1 | 100 |
| 17 | | | | | 1 | C | 5,0 | | | T | 1,0 | | 5,00 : 1 | 100 |
| 18 | | | | | 1 | C | 5,0 | | | R | 1,0 | | 5,00 : 1 | 100 |
| 19 | | | | | 1 | C | 5,0 | | | U | 1,0 | | 5,00 : 1 | 100 |
| 20 | 1 | | | | | C | 1,75 | | | Q | 1,25 | | 7,00 : 1 | 100 |

Tabelle 1 (Fortsetzung)

| Nr. | POLYHYDROXYVERBINDUNGEN | | | | | ORGANISCHE SÄUREHALOGENIDE | | | | | | MOLVERHÄLTNIS | | |
|---|---|---|---|---|---|---|---|---|---|---|---|---|---|---|
| | DHPVE 1) | TOB 2) | THB 3) | PHB 4) | HHB 5) | LSH 6) | | | | NLSH 7) | | $D_1/D_2$ | $D_1/D_0$ | VG 8) (%) |
| | MOL | | | | | $D_1$ | $Mol_{D_1}$ | $D_2$ | $Mol_{D_2}$ | $D_0$ | $Mol_{D_0}$ | | | |
| 21 | | 1 | | | | K | 2,4 | | | O | 0,3 | | 8,00 : 1 | 90 |
| 22 | | | 1 | | | C | 3,4 | M | 0,4 | | | 8,50 : 1 | | 95 |
| 23 | 1 | | | | | I | 1,7 | | | W | 0,2 | 8,50 : 1 | | 95 |
| 24 | | 1 | | | | G | 2,7 | | | N | 0,3 | | 9,00 : 1 | 100 |
| 25 | | 1 | | | | C | 2,7 | L | 0,3 | | | 9,00 : 1 | | 100 |
| 26 | | 1 | | | | C | 2,7 | M | 0,3 | | | 9,00 : 1 | | 100 |
| 27 | | | | 1 | | C | 4,0 | | | ST | 0,4 | | 10,0 : 1 | 88 |
| 28 | | | 1 | | | C | 3,75 | M | 0,25 | | | 15,0 : 1 | | 100 |
| 29 | 1 | | | | | C | 1,9 | | | S | 0,1 | | 19,0 : 1 | 100 |
| 30 | | | 1 | | | | | M | 4,0 | | | Vergl. | | 100 |
| 31 | | | 1 | | | | | L | 4,0 | | | Vergl. | | 100 |
| 32 | | | 1 | | | C | 4,0 | | | | | Vergl. | | 100 |
| 33 | | | | | 1 | C | 6,0 | | | | | Vergl. | | 100 |
| 34 | | | | | 1 | | | M | 6,0 | | | Vergl. | | 100 |
| 35 | | 1 | | | | | | M | 3,0 | | | Vergl. | | 100 |
| 36 | | | 1 | | | | | M | 4,0 | | | Vergl. | | 95 |

1) 4,4-Bis-(4 -hydroxyphenyl)-valeriansäure-ethoxyethylester (MG: 258)
2) 2,3,4-Trihydroxybenzophenon (MG: 230)
3) 2,3,4,4'-Tetrahydroxybenzophenon (MG: 246)
4) 2,2',3,4,4'-Pentahydroxybenzophenon (MG: 262)
5) 2,3,3',4,4',5-Hexahydroxybenzophenon (MG: 278)
6) Lichtempfindliches Säurehalogenid
7) Nichtlichtempfindliches Säurehalogenid
8) Veresterungsgrad

## Beispiel 2

Die Löslichkeit der photoaktiven Verbindungen wurde in einer 26-gew.-%igen Lösung eines Novolaks in Methoxypropanolacetat (MPA) bestimmt, da die entstehenden Lösungen der Realität eines Photoresistgemisches wesentlich mehr entsprechen als Lösungen der erfindungsgemäßen Verbindungen in reinem MPA. Dabei wurden die photoaktiven Verbindungen der Novolaklösung portionsweise zugesetzt bis keine vollständige Lösung mehr eintrat (bei den Vergleichsbeispielen) bzw. wurde die Zugabe bei bestimmten Prozentgehalten der Lösungen trotz weiter bestehender Löslichkeit abgebrochen (bei den erfindungsgemäßen Verbindungen). In allen Fällen konnte eine deutlich verbesserte Löslichkeit der erfindungsgemäßen Verbindungen gegenüber entsprechenden Vergleichsverbindungen festgestellt werden (siehe Tabelle 2).

Tabelle 2

| Verbindungen Nr. | Löslichkeit L (%) |
|---|---|
| 4 | >7,0 |
| 35 (Vergleich) | 2,4 <L< 4,8 |
| 5 | > 4,8 |
| 5a | > 7,0 |
| 9a | > 4,8 |
| 15 | > 7,0 |
| 30 (Vergleich) | < 2,4 |
| 32 (Vergleich) | < 2,4 |
| 6 | >11,1 |
| 36 (Vergleich) | 9,1 <L< 10,1 |
| 16 | 22,3 <L< 24,8 |
| 17 | >28,8 |
| 18 | >37,5 |
| 19 | 10,1 <L< 12,9 |
| 33 (Vergleich) | < 7,3 |
| 34 (Vergleich) | < 4,4 |

**Patentansprüche**

**1.** Strahlungsempfindlicher Ester aus

a) einer Verbindung mit zwei bis sechs aromatischen Hydroxygruppen,

b) einer kernsubstituierten o-Naphthochinon-2-diazid-4-sulfonsäure (Diazoverbindung $D_1$) und

c) einer nicht weiter substituierten o-Naphthochinon-2-diazid-4- bzw. -5-sulfonsäure (Diazoverbindung $D_2$) und/oder einer nicht strahlungsempfindlichen organischen Säure (Verbindung $D_0$),

wobei das molare Verhältnis von $D_1$:($D_2$ und/oder $D_0$) zwischen 0,1:1 und 39:1 liegt.

**2.** Strahlungsempfindlicher Ester gemäß Anspruch 1, dadurch gekennzeichnet, daß die Verbindung mit 2 bis 6 aromatischen Hydroxygruppen der allgemeinen Formel I entspricht

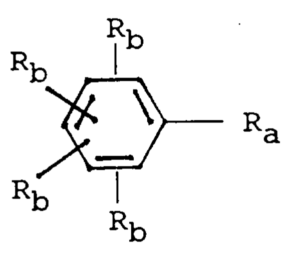

(I)

worin $R_a$ = -H, -X-$R_c$ oder

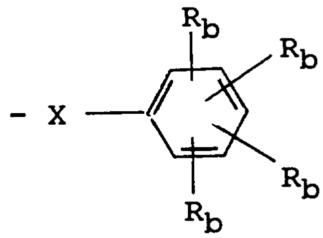

darstellt, worin

$R_b$ Wasserstoff- oder Halogenatome, Hydroxy- oder $(C_1$-$C_6)$Alkylgruppen bedeuten, mit der Maßgabe, daß mindestens zwei und höchstens sechs der $R_b$-Gruppen Hydroxygruppen und die Reste $R_b$ untereinander gleich oder verschieden sind,

X eine Einfachbindung, -O-, -S-, $-SO_2-$, -CO-, $-CO-CH_2-$, $-CO-CH_2-CH_2-$, $-CO-CH_2-CO-$, $-CO-CH_2-CH_2-CO-$, -CO-O-, $-CO-O-CH_2-$, $-CO-O-CH_2-CH_2-$, $-CH_2-CO-O-CH_2-$, $-CH_2-CO-O-CH_2-CH_2-$, $-CH_2-$, $-CH_2-CH_2-$, $-(CH_2)_3-$, $-C(CH_3)_2-$, $-C(CF_3)_2-$, $-CH_2-CH_2-Y$, $-CH_2-CH_2-CH_2-Y$, $-C(CH_3)-CH_2-Y$, $-C(CH_3)-CH_2-CH_2-Y$, wobei $CH_2$-Gruppen durch -O-, H-Atome durch Substituenten ersetzt sein können,

Y ein Wasserstoffatom oder eine gegebenenfalls substituierte Alkoxy-, Carboxy-, Alkoxycarbonyl-, Alkoxyalkoxycarbonyl- oder Arylgruppe und

$R_c$ ein Wasserstoffatom oder eine gegebenenfalls substituierte Alkyl- oder Arylgruppe bedeutet.

**3.** Strahlungsempfindlicher Ester gemäß Anspruch 1, dadurch gekennzeichnet, daß die Verbindung mit 2 bis 6 aromatischen Hydroxygruppen der allgemeinen Formel II entspricht

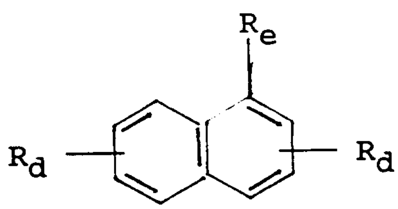

(II)

worin

$R_e$ ein Wasserstoffatom oder eine gegebenenfalls substituierte Naphthalin-1-ylmethylgruppe

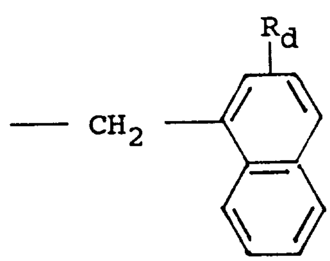

bedeutet, wobei

$R_d$ ein Wasserstoffatom oder eine Hydroxygruppe darstellt, mit der Maßgabe, daß mindestens zwei der $R_d$-Gruppen Hydroxygruppen sind.

**4.** Strahlungsempfindlicher Ester gemäß Anspruch 1, dadurch gekennzeichnet, daß die Verbindung mit 2 bis 6 aromatischen Hydroxygruppen der allgemeinen Formel III entspricht

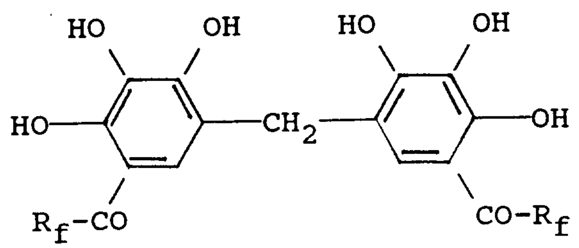

(III)

worin

$R_f$ ein Wasserstoffatom oder eine gegebenenfalls substituierte Alkyl-, Alkoxy- oder Arylgruppe bedeutet.

5. Strahlungsempfindlicher Ester gemäß Anspruch 1, dadurch gekennzeichnet, daß die kernsubstituierte o-Naphthochinon-2-diazid-4-sulfonsäure (Diazoverbindung $D_1$) der allgemeinen Formel IV entspricht

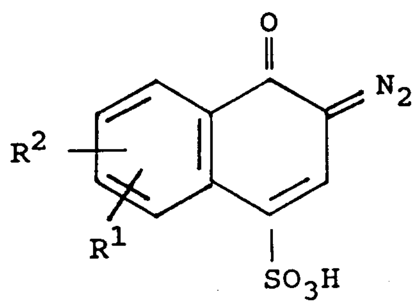

(IV)

worin

$R^1$ und $R^2$ gleich oder verschieden sind und ein Wasserstoffatom, eine Alkyl-, Alkoxy- oder Alkylmercaptogruppe, deren C-Ketten durch Sauerstoffatome unterbrochen sein können, eine Acylamino-, Alkoxycarbonyl-, N-Alkylsulfamoyl oder N,N-Dialkyl-sulfamoylgruppe bedeuten, mit der Maßgabe, daß

$R^1$ und $R^2$ nicht gleichzeitig Wasserstoff sind.

6. Strahlungsempfindlicher Ester gemäß Anspruch 1, dadurch gekennzeichnet, daß die o-Naphthochinon-2-diazid-4- bzw. -5-sulfonsäure (Diazoverbindung $D_2$) der allgemeinen Formel V entspricht

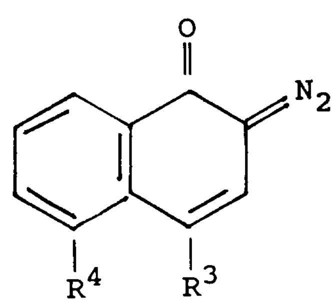

(V)

worin

$R^3$ und $R^4$ verschieden sind und ein Wasserstoffatom oder eine Sulfonylgruppe bedeuten.

7. Strahlungsempfindlicher Ester gemäß Anspruch 1, dadurch gekennzeichnet, daß die nicht strahlungsempfindliche organische Säure $D_0$ eine Verbindung der allgemeinen Formel VI

$R^5$ - Z - OH (VI)

ist, worin

$R^5$ eine gesättigte oder ungesättigte, geradkettige oder verzweigte $(C_1$-$C_{25})$Alkyl-, $(C_1$-$C_{25})$-Alkoxy- oder $(C_1$-$C_{25})$Alkylmercaptogruppe, deren C-Ketten durch Sauerstoffatome unterbrochen oder die substituiert sein können, eine gegebenenfalls substituierte $(C_6$-$C_{14})$Aryl-, $(C_6$-$C_{14})$Aralkyl- oder $(C_6$-$C_{14})$Cycloalkylgruppe und

Z eine Carbonyl- oder Sulfonylgruppe

bedeuten.

**8.** Strahlungsempfindlicher Ester gemäß Anspruch 5, dadurch gekennzeichnet, daß $R^1$ ein Wasserstoffatom und $R^2$ eine Alkyl-, Alkoxy- oder Alkylmercaptogruppe ist, deren C-Ketten durch Sauerstoffatome unterbrochen sein können.

**9.** Strahlungsempfindlicher Ester gemäß Anspruch 8, dadurch gekennzeichnet, daß $R^1$ ein Wasserstoffatom und $R^2$ eine 7-Methoxygruppe ist.

**10.** Strahlungsempfindlicher Ester gemäß Anspruch 2, dadurch gekennzeichnet, daß die aromatische Polyhydroxyverbindung ein Tri-, Tetra-, Penta- oder Hexahydroxybenzophenon oder ein Trihydroxyphenyl-alkylketon ist.

**11.** Strahlungsempfindlicher Ester gemäß Anspruch 1, dadurch gekennzeichnet, daß das Molverhältnis von $D_1$:($D_2$ und/oder $D_0$) zwischen 0,25:1 und 19:1, insbesondere zwischen 1,5:1 und 9:1, liegt.

## Claims

**1.** A radiation-sensitive ester composed of

a) a compound containing two to six aromatic hydroxyl groups,

b) a ring-substituted (o-naphthoquinone 2-diazide)-4-sulfonic acid (diazo compound $D_1$), and

c) an (o-naphthoquinone 2-diazide)-4- or -5-sulfonic acid which is not further substituted (diazo compound $D_2$) and/or a non-radiation-sensitive organic acid (compound $D_0$),

where the $D_1$:($D_2$ and/or $D_0$) molar ratio is between 0.1:1 and 39:1.

**2.** A radiation-sensitive ester as claimed in claim 1, wherein the compound containing 2 to 6 aromatic hydroxyl groups corresponds to the formula I

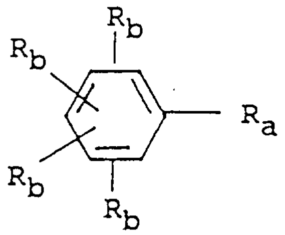

(I)

where $R_a$ = -H, -X-$R_c$ or

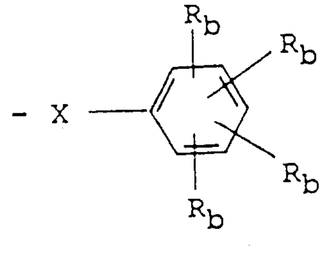

in which

$R_b$ is a hydrogen or halogen atom, or a hydroxyl or $(C_1$-$C_6)$ alkyl group, with the proviso that at

least two and not more than six of the $R_b$ groups are hydroxyl groups and the radicals $R_b$ are identical to, or different from, one another,

X is a single bond, -O-, -S-, $-SO_2-$, -CO-, $-CO-CH_2-$, $-CO-CH_2-CH_2-$ $-CO-CH_2-CO-$, $-CO-CH_2-CH_2-CO-$, -CO-O-, $-CO-O-CH_2-$, $-CO-O-CH_2-CH_2-$, $-CH_2-CO-O-CH_2-$, $-CH_2-CO-O-CH_2-CH_2-$, $-CH_2-$, $-CH_2-CH_2-$, $-(CH_2)_3-$, $-C(CH_3)_2-$, $-C(CF_3)_2-$, $-CH_2-CH_2-Y$, $-CH_2-CH_2-CH_2-Y$, $-C(CH_3)-CH_2-Y$, $-C(CH_3)-CH_2-CH_2-Y$, where $CH_2$ groups may be replaced by -O-, and hydrogen atoms by substituents,

Y is a hydrogen atom or an optionally substituted alkoxy, carboxyl, alkoxycarbonyl, alkoxyalkoxycarbonyl or aryl group, and

$R_c$ is a hydrogen atom or an optionally substituted alkyl or aryl group.

**3.** A radiation-sensitive ester as claimed in claim 1, wherein the compound containing 2 to 6 aromatic hydroxyl groups corresponds to the formula II

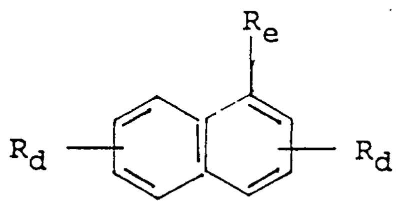

(II)

in which

$R_e$ is a hydrogen atom or an optionally substituted 1-naphthylmethyl group

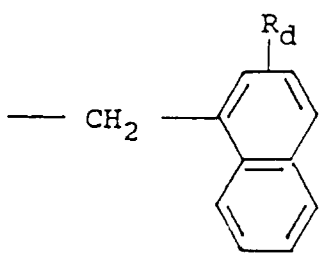

where

$R_d$ is a hydrogen atom or a hydroxyl group, with the proviso that at least two of the $R_d$ groups are hydroxyl groups.

**4.** A radiation-sensitive ester as claimed in claim 1, wherein the compound containing 2 to 6 aromatic hydroxyl groups corresponds to the formula III

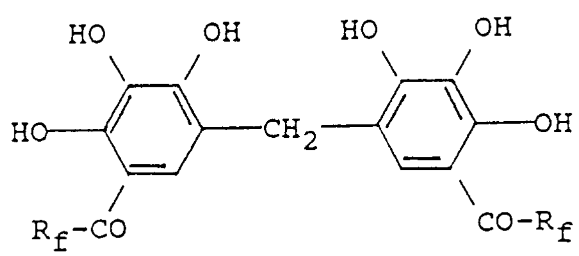

(III)

in which

$R_f$ is a hydrogen atom or an optionally substituted alkyl, alkoxy or aryl group.

5. A radiation-sensitive ester as claimed in claim 1, wherein the ring-substituted (o-naphthoquinone 2-diazide)-4-sulfonic acid (diazo compound $D_1$) corresponds to the formula IV

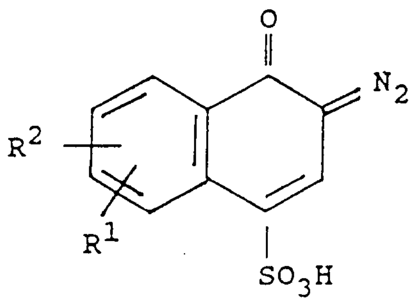

(IV)

in which

$R^1$ and $R^2$ are identical or different and are a hydrogen atom, an alkyl, alkoxy or alkylmercapto group whose carbon chains may be interrupted by oxygen atoms, an acylamino, alkoxycarbonyl, N-alkylsulfamoyl or N,N-dialkylsulfamoyl group, with the proviso that

$R^1$ and $R^2$ are not hydrogen at the same time.

6. A radiation-sensitive ester as claimed in claim 1, wherein the (o-naphthoquinone 2-diazide)-4- or -5-sulfonic acid (diazo compound $D_2$) corresponds to the formula V

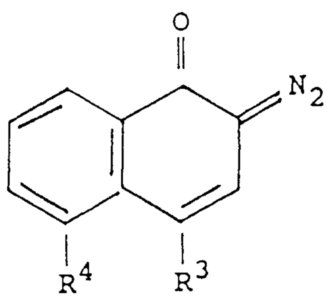

(V)

in which

$R^3$ and $R^4$ are different and are a hydrogen atom or a sulfonyl group.

7. A radiation-sensitive ester as claimed in claim 1, wherein the non-radiation-sensitive organic acid $D_0$ is a compound of the formula VI

$R^5$ - Z - OH (VI)

in which

$R^5$ is a saturated or unsaturated, straight-chain or branched ($C_1$-$C_{25}$)alkyl, ($C_1$-$C_{25}$)alkoxy or ($C_1$-$C_{25}$)alkylmercapto group whose carbon chains may be interrupted by oxygen atoms or which may be substituted, an optionally substituted ($C_6$-$C_{14}$)aryl, ($C_6$-$C_{14}$)aralkyl or ($C_6$-$C_{14}$)-cycloalkyl group, and

Z is a carbonyl or sulfonyl group.

8. A radiation-sensitive ester as claimed in claim 5, wherein $R^1$ is a hydrogen atom and $R^2$ is an alkyl, alkoxy or alkylmercapto group whose carbon chains may be interrupted by oxygen atoms.

9. A radiation-sensitive ester as claimed in claim 8, wherein $R^1$ is a hydrogen atom and $R^2$ is a 7-methoxy group.

**10.** A radiation-sensitive ester as claimed in claim 2, wherein the aromatic polyhydroxyl compound is a tri-, tetra-, penta- or hexahydroxybenzophenone or a trihydroxyphenyl alkyl ketone.

**11.** A radiation-sensitive ester as claimed in claim 1, wherein the $D_1$:($D_2$ and/or $D_0$) molar ratio is between 0.25:1 and 19:1, in particular between 1.5:1 and 9:1.

**Revendications**

**1.** Ester photosensible constitué de
a) un composé présentant de deux à six groupes hydroxyles aromatiques,
b) un acide o-naphtoquinone-2-diazide-4-sulfonique substitué sur le noyau (composé diazo $D_1$) et
c) un acide o-naphtoquinone-2-diazide-4-respectivement -5-sulfonique ne présentant pas d'autre substituant (composé diazo $D_2$) et/ou un acide organique non photosensible (composé $D_0$),
dans lequel le rapport molaire de $D_1$ à ($D_2$ et/ou $D_0$) est situé entre 0,1:1 et 39:1.

**2.** Ester photosensible selon la revendication 1, caractérisé en ce que le composé présentant de deux à six groupes hydroxyles aromatiques correspond à la formule générale I

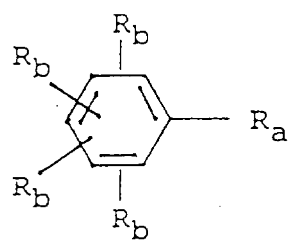

(I)

dans laquelle $R_a$ représente -H, -X-$R_c$ ou

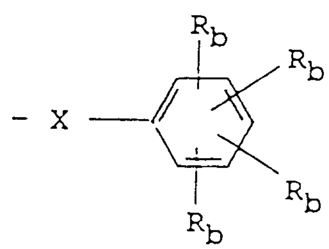

dans laquelle,

$R_b$ identiques ou différents, représentent des atomes d'hydrogène ou bien d'halogène ou des groupes hydroxyle ou $C_1$-$C_6$-alkyle à la condition qu'au moins deux et au plus six des groupes $R_b$ soient des groupes hydroxyles,

X représent une liaison simple,-O-, -S-, $-SO_2-$, -CO-, $-CO-CH_2-$, $-CO-CH_2-CH_2-$, $-CO-CH_2-CO-$, $-CO-CH_2-CH_2-CO-$, -CO-O-, $-CO-O-CH_2-$, $-CO-O-CH_2-CH_2-$, $-CH_2-CO-O-CH_2-$, $-CH_2-CO-O-CH_2-CH_2-$, $-CH_2-$, $-CH_2-CH_2-$, $-(CH_2)_3-$, $-C(CH_3)_2-$, $-C(CF_3)_2-$, $-CH_2-CH_2-Y$, $-CH_2-CH_2-CH_2-Y$, $-C(CH_3)-CH_2-Y$, $-C(CH_3)-CH_2-CH_2-Y$, dans lesquels les groupes $CH_2$ peuvent être remplacés par -O-, et les atomes d'hydrogène peuvent être remplacés par des substituants,

Y représente un atome d'hydrogène ou un groupe alcoxy, carboxy, alcoxycarbonyle, alcoxyalcoxycarbonyle, ou aryle, éventuellement substitué, et

$R_c$ représente un atome d'hydrogène ou un groupe alkyle ou aryle éventuellement substitué.

**3.** Ester photosensible selon la revendication 1, caractérisé en ce que le composé présentant de deux à six groupes hydroxyles aromatiques correspond à la formule générale II

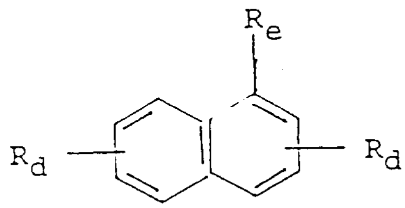

(II)

dans laquelle

$R_e$ représente un atome d'hydrogène ou un groupe napht-1-yl-méthyle éventuellement substitué

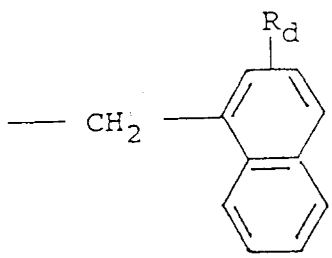

dans laquelle

$R_d$ représente un atome d'hydrogène ou un groupe hydroxyle, à la condition qu'au moins deux des groupes $R_d$ soient des groupes hydroxyles.

**4.** Ester photosensible selon la revendication 1, caractérisé en ce que le composé présentant de deux à six groupes hydroxyles aromatiques correspond à la formule générale III

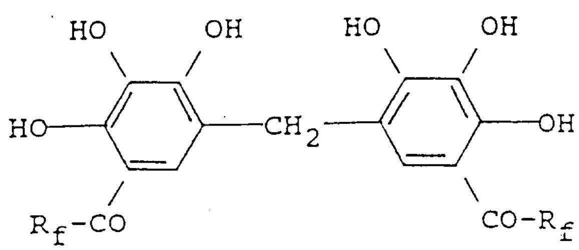

(III)

dans laquelle

$R_f$ représente un atome d'hydrogène ou un groupe alkyle, alcoxy ou aryle éventuellement substitué.

**5.** Ester photosensible selon la revendication 1, caractérisé en ce que l'acide o-naphtoquinone-2-diazide-4-sulfonique substitué sur le noyau (composé diazo $D_1$) correspond à la formule générale IV

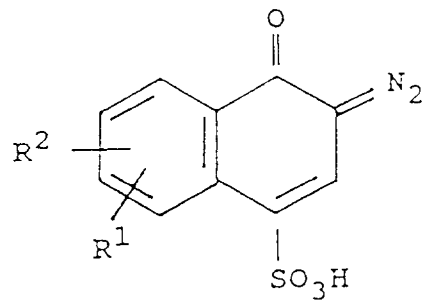

(IV)

dans laquelle

$R^1$ et $R^2$ identiques ou différents, représentent un atome d'hydrogène, un groupe alkyle, alcoxy ou alkylmercapto, dont les chaînes carbonées peuvent être interrompues par des atomes d'oxygène, un groupe acylamino, alcoxycarbonyle, N-alkylsulfamoyle ou N,N-dialkyl-sulfamoyle, à la condition que

$R^1$ et $R^2$ ne soient pas simultanément un atome d'hydrogène.

**6.** Ester photosensible selon la revendication 1, caractérisé en ce que l'acide o-naphtoquinone-2-diazide-4- respectivement -5-sulfonique (composé $D_2$) correspond à la formule générale V

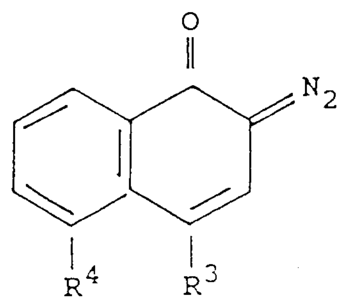

(V)

dans laquelle

$R^3$ et $R^4$ sont différents et représentent un atome d'hydrogène ou un groupe sulfonyle.

**7.** Ester photosensible selon la revendication 1, caractérisé en ce que l'acide organique non photosensible $D_0$ est un composé de formule générale VI

$R^5$ - Z - OH (VI)

dans laquelle

$R^5$ représente une chaîne saturée ou insaturée, linéaire ou ramifiée de type $(C_1\text{-}C_{25})$alkyle, $(C_1\text{-}C_{25})$alcoxy ou $(C_1\text{-}C_{25})$alkylmercapto, dont les chaînes carbonées peuvent être éventuellement interrompues par des atomes d'oxygène ou peuvent être subsituées, un groupe $(C_6\text{-}C_{14})$aryle, $(C_6\text{-}C_{14})$aralkyle ou $(C_6\text{-}C_{14})$cycloalkyle, éventuellement substitué et

Z un groupe carbonyle ou sulfonyle.

**8.** Ester photosensible selon la revendication 5, caractérisé en ce que $R^1$ est un atome d'hydrogène et $R^2$ est un groupe alkyle, alcoxy ou alkylmercapto, dont les chaînes carbonées peuvent être éventuellement interrompues par des atomes d'oxygène.

**9.** Ester photosensible selon la revendication 8, caractérisé en ce que $R^1$ est un atome d'hydrogène et $R^2$ est un groupe 7-méthoxy.

10. Ester photosensible selon la revendication 2, caractérisé en ce que le composé aromatique polyhydroxylé est une tri-, tétra-, penta- ou hexahydroxybenzophénone ou une tri-hydroxyphényl-alkylcétone.

11. Ester photosensible selon la revendication 1, caractérisé en ce que le rapport molaire de $D_1$ à ($D_2$ et/ou $D_0$) est situé entre 0,25:1 et 19:1, plus particulièrement entre 1,5:1 et 9:1.